(19) Europäisches Patentamt
European Patent Office
Office européen des brevets

(11) Publication number: **0 075 392 A1**

(12) **EUROPEAN PATENT APPLICATION**

(21) Application number: **82304434.2**

(22) Date of filing: **23.08.82**

(51) Int. Cl.³: **C 07 H 21/00**

(30) Priority: **24.08.81 US 295420**
**24.08.81 US 295421**

(43) Date of publication of application: **30.03.83**
**Bulletin 83/13**

(84) Designated Contracting States: **BE CH DE FR GB IT LI LU NL SE**

(71) Applicant: **ELI LILLY AND COMPANY, 307, East McCarty Street, Indianapolis Indiana 46285 (US)**

(72) Inventor: **Hsiung, Hansen Maxwell, 108 West 88th Street, Indianapolis Indiana 46260 (US)**

(74) Representative: **Hudson, Christopher Mark et al, Erl Wood Manor, Windlesham Surrey GU20, 6PH (GB)**

(54) **Process for de-cyanoethylating blocked nucleotides.**

(57) A process for selectively removing a β-cyanoethyl blocking group from the phosphate moiety of a nucleotide or polynucleotide, which comprises treating said nucleotide or polynucleotide with a primary amine having from 3 to 5 carbon atoms or diethylamine.

EP 0 075 392 A1

Title

PROCESS FOR DE-CYANOETHYLATING BLOCKED NUCLEOTIDES

This invention concerns a process for selectively removing a β-cyanoethyl blocking group from the phosphate moiety of a nucleotide or polynucleotide by treatment with a primary amine having 3 to 5 carbon atoms or diethylamine which is useful in the synthesis of oligonucleotides of defined sequences.

With the advent of recombinant DNA methodology and especially in view of its evident commercial applicability, the ability to synthesize oligodeoxyribonucleotides of defined sequences has become increasingly important.

As now is very well recognized, RNA and DNA are polynucleotides referred to as nucleic acids. The polynucleotides, in turn, are composed of monomers (nucleotides). A nucleotide is a phosphate ester of the N-glycoside of a nitrogenous base and consists of a purine or a pyrimidine base, a pentose (D-ribose in RNA or 2'-deoxy-D-ribose in DNA), and a phosphate group.

Four nitrogenous bases are present in both DNA and RNA. The four present in DNA are:

BAD ORIGINAL

Adenine (A)

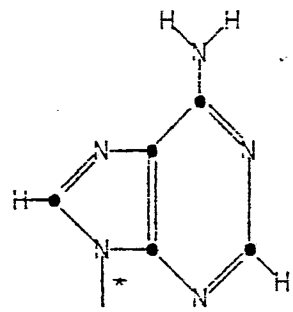

Guanine (G)

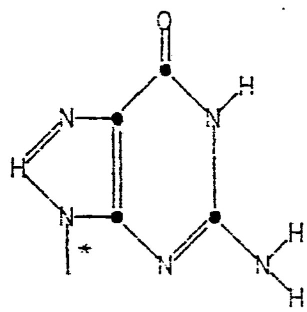

Thymine (T)

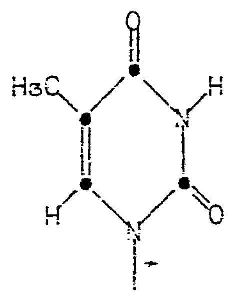

Cytosine (C)

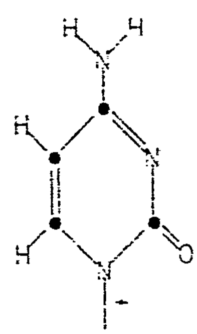

BAD ORIGINAL

The nitrogenous bases in RNA differ from those in DNA only in that uracil (U) replaces thymine.

Uracil (U)

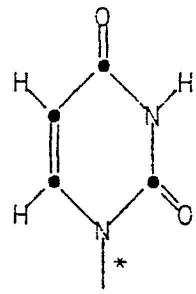

The combination of a nitrogenous base at the point of the asterisk (*) in the foregoing formulas with a ribose at its 1'-position is called a ribonucleoside (D-ribose) or a deoxyribonucleoside (2'-deoxy-D-ribose). The corresponding ribonucleotide or deoxyribonucleotide is produced by addition of a phosphate group at the 3'-position of the ribose.

The thus-defined suitably-blocked ribonucleotide or deoxyribonucleotide represents the basic building block in the synthesis of RNA or DNA, respectively. A standard and highly attractive method for synthesizing RNA or DNA is known in the literature as the "triester method". Using the synthesis of a polydeoxyribonucleotide as an example, the procedure involves coupling a mononucleotide or oligonucleotide having a 3'-phosphate diester with a mononucleoside, a blocked 3'-hydroxy oligonucleotide, a mononucleotide, or an oligonucleotide having an available 5'-hydroxyl group. This method can be represented schematically as follows:

BAD ORIGINAL

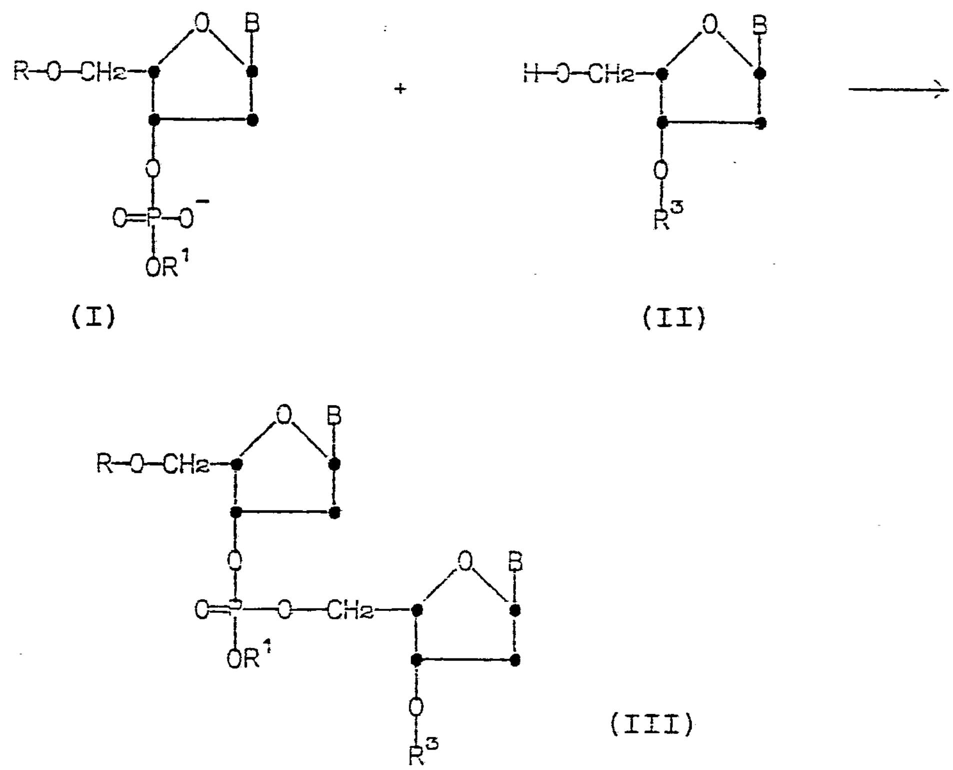

+

(I) (II)

(III)

In the foregoing, B is a nitrogenous base selected from adenine, cytosine, guanine, and thymine, each of the first three having their reactive moieties blocked by suitable protecting groups; R is a blocking group for the 5'-hydroxyl; $R^3$ is a blocking group for the 3'-hydroxyl cleavable under alkaline conditions or a group of the formula

$$O=\overset{|}{P}-OR^2 \quad ;$$
$$\;\;\;\;\overset{|}{O}R^1$$

and $R^1$ and $R^2$ are selectively removable groups which block the reactive phosphate moiety.

Additional discussion of the triester method can be found in various publications including, for example, Narang, S.A., Hsiung, H.M., and Brousseau, R.,

BAD ORIGINAL

"Improved Phosphotriester Method for the Synthesis of Gene Fragments", Methods in Enzymology, Vol. 68, Academic Press, New York, New York, (1979), pp. 90-98; and Narang, S.A., Brousseau, R., Hsiung, H.M., and Michniewicz, J.J., "Chemical Synthesis of Deoxyoligonucleotides by the Modified Triester Method", Methods in Enzymology, Vol. 65, Academic Press, New York, New York, (1980), pp. 610-620.

The triester method described above, of course, has been applied in coupling two oligonucleotides, an oligonucleotide and a mononucleotide, or, as specifically illustrated above, two mononucleotides. Whatever the entities, the reaction involves the coupling of an available 5'-hydroxyl with a 3'-phosphate diester group. Moreover, the reactant containing the available 5'-hydroxyl can have a blocked terminal phosphate or such can be lacking (i.e., $R^3$ is a 3'-hydroxyl blocking group). The two reactants are coupled in the presence of a solvent, typically pyridine, and in the presence of a coupling agent, for example, 2,4,6-trimethylbenzenesulfonyl tetrazolide.

Whatever the structure of the particular nucleotide reactants, it is essential that the reactant having an available 5'-hydroxyl group be fully blocked at the terminal 3'-phosphate group if such is present. The group $R^2$ appearing in the foregoing formulas represents a blocking group that completes protection of the terminal 3'-phosphate group. If further coupling of the product polynucleotide at its 3'-phosphate site is intended, it is important that the $R^2$ group be one that is removable without disruption of other blocking groups on the molecule.

BAD ORIGINAL

Customarily, the $R^2$ group of choice is β-cyanoethyl, a group which is selectively removable under mild alkaline conditions. A highly preferred method for selectively removing a β-cyanoethyl group from a nucleotide or oligonucleotide uses anhydrous triethylamine-pyridine. This method is reported in Adamiak, R.W., Barciszewska, M.Z., Biala, E., Grzeskowiak, K., Kierzek, R., Kraszewski, A., Markiewicz, W.T., and Wiewiorowski, M., Nucleic Acids Research 3, 3397-3408 (1976), and Sood, A.K., and Narang, S.A., Nucleic Acids Research 4, 2757-2765 (1977). Although this method has been highly successful in achieving selective elimination of the β-cyanoethyl blocking group, it suffers from the fact that it is time-consuming, taking 4-6 hours to complete (see Sood et al., supra, p. 2758).

There has now been discovered a process for selectively removing a β-cyanoethyl blocking group from the phosphate moiety of a nucleotide or polynucleotide, which comprises treating said nucleotide or polynucleotide with a primary amine having from 3 to 5 carbon atoms or diethylamine.

As noted, this invention is directed to a process for facilitating selective removal of a β-cyanoethyl blocking group from the phosphate moiety of a nucleotide or polynucleotide.

In any polynucleotide preparation, suitable blocking of otherwise reactive moieties is essential. Thus, certain of the nitrogenous bases require blocking of their reactive amino groups. Typically, adenine and cytosine are benzoylated to protect their free amino groups, and guanine customarily is protected by an

BAD ORIGINAL

isobutyryl group. Thymine, since it has no free amino group, requires no protection. Of course, the foregoing are only examples of suitable blocking groups. Any of a wide range of other blocking groups can be employed.

The group R in the foregoing formulas represents a protecting group for the 5'-hydroxyl moiety. Preferably, the group is labile under mildly acidic conditions. Examples of such groups are tetrahydropyrenyl, 4-methoxytrityl, 4,4'-dimethoxytrityl (DMTr), and the like. 4,4'-Dimethoxytrityl is readily removed in mild acid, for example, 2% benzenesulfonic acid, and represents a preferred protecting group for the 5'-hydroxyl moiety.

The "triester method" is so-called because the coupled product is a phosphate triester. This, in turn, contemplates the use of a nucleotide reactant having a partially-blocked 3'-phosphate group. The group $R^1$ used herein is intended to refer to a group that completes a suitable partial block. This group preferably is one that is removable under alkaline conditions. Examples of suitable groups are phenyl, *o*-chlorophenyl, 2,4-dichlorophenyl, *p*-chlorophenyl, *p*-nitrophenyl, *o*-nitrophenyl, 2,4-dinitrophenyl, *p*-mercaptophenyl, and the like. Preferably, $R^1$ is *p*-chlorophenyl.

A fully blocked nucleotide or the 3'-phosphate-containing terminal of a polynucleotide produced by the triester method contains a blocked phosphate of the formula

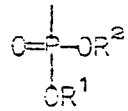

BAD ORIGINAL

The group $R^2$ represents a group that can be selectively cleaved, generally under mild alkaline conditions and, in accordance with the process of this invention, is β-cyanoethyl. A blocked phosphate in which $R^1$ is p-chlorophenyl and $R^2$ is β-cyanoethyl is conveniently denoted OPCE.

It is also possible that the 3'-terminal of a polynucleotide product lacks a phosphate in which case $R^3$, as hereinbefore described, is a 3'-hydroxyl blocking group cleavable under alkaline conditions. Suitable such groups are acetyl, benzoyl, p-methoxybenzoyl, and chloroacetyl. Preferably, the blocking group is benzoyl (Bz).

Nucleotide coupling is carried out in the presence of a coupling agent. Suitable coupling agents are well recognized by those skilled in the art. Examples of suitable coupling agents are p-nitrobenzenesulfonyl triazolide, benzenesulfonyl triazolide, benzenesulfonyl 4-nitroimidazolide, 2,4,6-triisopropylbenzenesulfonyl 3-nitro-1,2,4-triazolide, 2,4,6-trimethylbenzenesulfonyl 3-nitro-1,2,4-triazolide, 1-(p-toluenesulfonyl) 4-nitroimidazolide, 2,4,6-trimethylbenzenesulfonyl tetrazolide, 2,4,6-triisopropylbenzenesulfonyl chloride, 2,4,6-trimethylbenzenesulfonyl chloride, 2,4,6-triisopropylbenzenesulfonyl tetrazolide, and the like. Preferably, the coupling agent is 2,4,6-triisopropylbenzenesulfonyl tetrazolide or 2,4,6-trimethylbenzenesulfonyl tetrazolide.

The coupling reaction is carried out in the presence of a suitable inert organic solvent under substantially anhydrous conditions. The current solvent of choice is pyridine.

BAD ORIGINAL

Products produced by nucleotide coupling include polynucleotides having the formula

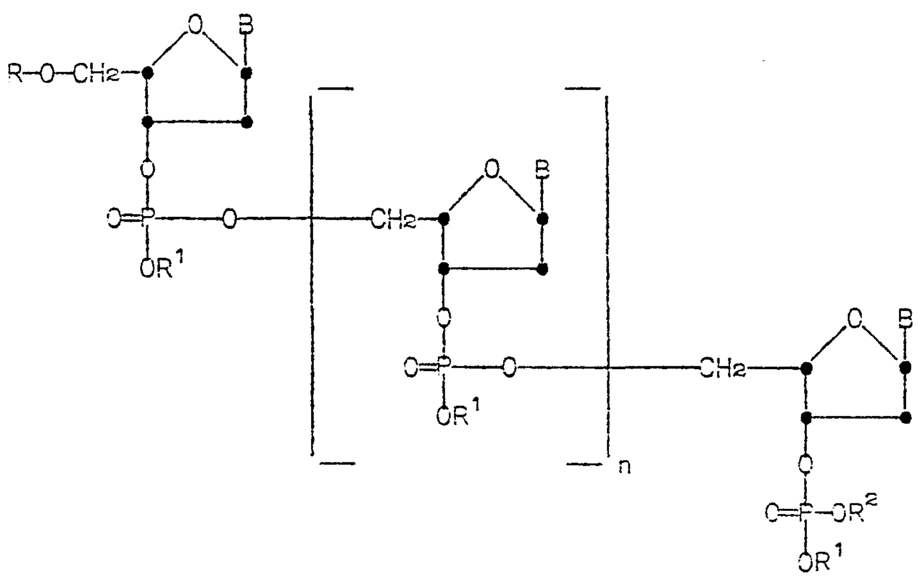

in which B, R, $R^1$, and $R^2$ are as hereinbefore defined and n is zero or an integer up to about 20.

These as well as fully blocked mononucleotides of the formula

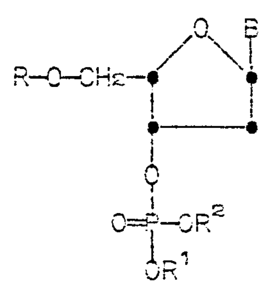

represent the kinds of structures to which the selective deblocking process of this invention may be applied.

BAD ORIGINAL

In deblocking the β-cyanoethyl ($R^2$) group of a mono- or polynucleotide in accordance with the process of this invention, the mono- or polynucleotide is reacted with a primary amine having from 3 to 5 carbon atoms or with diethylamine. Examples of such primary amines are n-propyl amine, n-butyl amine, sec-butyl amine, and t-butyl amine. A hindered amine is highly preferred, and, in particular, t-butyl amine.

Although not essential, the deblocking reaction generally and preferably is carried out in the presence of an inert organic solvent. The current solvent of choice is pyridine.

For the sake of convenience, the decyanoethylation reaction normally is carried out at room temperature. However, any of a wide range of temperatures, generally ranging from 4°C. to 30°C. can be employed.

The amount of primary amine or diethylamine used in the process of this invention is directly dependent upon the amount, on a molar basis, of β-cyanoethyl to be cleaved. However, since an excess of the amine can be used without detriment, a large excess, for example, 10 to 20 fold, on a molar basis normally will be used.

The progress of the reaction can be monitored using any of a variety of analytical techniques including, for example, thin-layer chromatography (tlc), high performance liquid chromatography (hplc), and the like.

The product, the amine salt of the phosphate diester, can be readily isolated by evaporation of the solvent, any excess amine, and the acrylonitrile by-

BAD ORIGINAL

product. If desired, the product can be further purified, for example, by recrystallization from a suitable solvent or solvent mixture. The recovered product, with or without further purification, is available for further coupling with a nucleotide or polynucleotide having an available 5'-hydroxyl.

The following examples are provided to further illustrate the process of this invention. They are not intended to be limiting upon the scope thereof.

Example 1 --Synthesis of DMTrO-TG-OPCE.

5'-O-Dimethoxytritylthymidine-3'-O-p-chlorophenyl-β-cyanoethyl phosphate (1.5 g. 1.9 mmol.) was dissolved in 20 ml. of anhydrous pyridine. t-Butylamine (about 3 ml.) was added dropwise to the above solution with stirring. The reaction was allowed to proceed for about 10 minutes after which decyanoethylation was complete as shown by silica gel thin-layer chromatography (solvent: 10% $MeOH/CH_2Cl_2$).

The reaction mixture was evaporated to dryness to remove pyridine, t-butylamine, and acrylonitrile. The dried reaction mixture was then redissolved in $CH_2Cl_2$ (20 ml.), and N-isobutyryldeoxyguanosine-3'-O-p-chlorophenyl-β-cyanoethyl phosphate (0.93 g., 1.6 mmol.) was added. The two protected mononucleotides were dried extensively *in vacuo* and redissolved in 10 ml. of anhydrous pyridine. The condensing agent, 2,4,6-trimethylbenzenesulfonyl tetrazolide (1.01 g., 4 mmol.), was added, and the reaction was allowed to proceed for one hour at ambient temperature. At the end of this period, the coupling reaction was shown to

BAD ORIGINAL

be complete by the disappearance of the 5'-hydroxyl reactant and appearance of an intense trityl positive spot of the dinucleotide (tlc: 10% $MeOH/CH_2Cl_2$).

The reaction mixture then was pipetted and transferred to 150 ml. of a 1:1 volume mixture of ether and hexane. A white precipitate containing the dinucleotide product formed and was collected by centrifugation. Thin-layer chromatography (10% $MeOH/CH_2Cl_2$) of the supernatant showed no dinucleotide product.

Example 2 --Synthesis of DMTrO-GGTA-OPCE.

To 200 mg. of DMTrO-GG-OPCE were added approximately 4 ml. of dry pyridine and approximately 2 ml. of t-butyl amine. The decyanoethylation was complete after 10 minutes, as determined by tlc, and the mixture was evaporated to dryness *in vacuo*. The decyanoethylated dimer was combined with $1.21 \times 10^{-4}$ moles of HO-TA-OPCE, and the mixture was dried for 30 minutes *in vacuo*. To initiate coupling, 200 mg. of 2,4,6-trimethylbenzenesulfonyl tetrazolide and approximately 4 ml. of dry pyridine were added. Coupling was complete after one hour. The mixture then was stirred into two centrifuge tubes, each containing 40 ml. of ethyl ether. A precipitate of the tetramer formed. The tubes were centrifuged for four minutes, the ether was decanted, and the product was dissolved in dichloromethane, and the solution was evaporated to dryness. After purification on a silica plate in 25% acetone, 70% ethyl acetate, 5% water, 121 mg. (43.2%) of the title compound were recovered.

BAD ORIGINAL

Example 3 --Synthesis of DMTrO-CGGACAGAGACA-OBz.

To 56 mg. of DMTrO-CGGA-OPCE were added 2 ml. of pyridine and 1 ml. of t-butyl amine. After 10 minutes the reaction mixture was dried in vacuo. To the residue were added 52 mg. of HO-CAGAGACA-OBz, and the mixture was dried in vacuo for 30 minutes. To the mixture then were added 90 mg. of 2,4,6-trimethylbenzenesulfonyl tetrazolide and 0.5 ml. of dry pyridine. The coupling reaction was completed after one hour. The mixture then was stirred into a centrifuge tube containing 40 ml. of ethyl ether. After centrifuging for four minutes, the ether was decanted, and the product was dissolved in dichloromethane and, the solution was evaporated to dryness. Purification of 5/6 of the material on a silica plate in 25% acetone, 70% ethyl acetate, 5% water gave 40 mg. of the title compound (57.3% yield based upon purification of all of the product).

Example 4 --Decyanoethylation of DMTrO-C-OPCE.

5'-O-Dimethoxytrityl-N'-benzoyldeoxycytidine-3'-O-p-chlorophenyl-β-cyanoethyl phosphate (170 mg., 0.2 mmol.) was dissolved in 0.2 ml. of anhydrous pyridine. About 1 ml. of sec-butylamine was added to the solution dropwise. The decyanoethylation was complete in 20 minutes as shown by silica gel chromatography. Excess sec-butylamine and pyridine along with acrylonitrile by-product were removed in vacuo, and the decyanoethylated product was isolated quantitatively.

BAD ORIGINAL

Example 5 --Decyanoethylation of DMTrO-T-OPCE.

5'-O-Dimethoxytritylthymidine-3'-O-p-chlorophenyl-β-cyanoethyl phosphate (150 mg., 0.19 mmol.) was dissolved in 2 ml. of anhydrous pyridine. About 1 ml. of n-propylamine was added to the solution. Decyanoethylation was complete after about 2 minutes as shown by tlc (silica gel GF; 10% $CH_3CH:CH_2Cl_2$). The reaction was quenched immediately by precipitation in a 2:1 mixture of hexane and ether. The resulting mixture was centrifuged, and the supernatant was decanted. The decyanoethylated product was condensed with N-benzoyldeoxyadenosine-3'-p-chlorophenyl-β-cyanoethyl phosphate as in the foregoing examples to produce DMTrO-TA-OPCE.

Example 6 --Synthesis of DMTrO-TA-OPCE.

5'-O-Dimethoxytritylthymidine-3'-O-p-chlorophenyl-β-cyanoethyl phosphate (3.0 g. 3.8 mmol.) was dissolved in 20 ml. of anhydrous pyridine. Diethylamine (about 6 ml.) was added dropwise to the above solution with stirring. The reaction was allowed to proceed for about 30 minutes after which decyanoethylation was complete as shown by silica gel thin-layer chromatography (solvent: 10% $MeOH/CH_2Cl_2$).

The reaction mixture was evaporated to dryness to remove pyridine, diethylamine, and acrylonitrile. The dried reaction mixture was then redissolved in $CH_2Cl_2$ (20 ml.), and N-benzoyldeoxyadenosine-3'-O-p-chlorophenyl-β-cyanoethyl phosphate (2.0 g., 3.2 mmol.) was added. The two protected mononucleotides were dried extensively in vacuo and redissolved in 20 ml. of anhydrous pyridine. The condensing agent, 2,4,6-

BAD ORIGINAL

trimethylbenzenesulfonyl tetrazolide (3.0 g., 12 mmol.), was added, and the reaction was allowed to proceed for one hour at ambient temperature. At the end of this period, the coupling reaction was shown to be complete by the disappearance of the 5'-hydroxyl reactant and appearance of an intense trityl positive spot of the dinucleotide (tlc: 10% $MeOH/CH_2Cl_2$).

The reaction mixture then was pipetted and transferred to 150 ml. of a 1:1 volume mixture of ether and hexane. A white precipitate containing the fully protected TA dimer in a yield of about 71% was collected by centrifugation. Thin-layer chromatography (10% $MeOH/CH_2Cl_2$) of the supernatant showed no dinucleotide product.

Example 7 --Synthesis of DMTrO-GTTC-OPCE.

To 300 mg. of DMTrO-GT-OPCE were added approximately 2 ml. of dry pyridine. Neat diethylamine (approximately 2 ml.) was added, and decyanoethylation was complete in less than 30 minutes, as determined by tlc. The mixture was evaporated to dryness <u>in vacuo</u>. The decyanoethylated dimer was reacted with 200 mg. of HO-TC-OPCE using the procedure of Example 6 to obtain 260 mg. (79%) of the title compound.

Example 8 --Synthesis of DMTrO-GTTCTACT-OBz.

To 260 mg. of DMTrO-GTTC-OPCE were added 2 ml. of pyridine. Neat diethylamine (approximately 1 ml.) was added, and decyanoethylation was complete in less than 30 minutes, as determined by tlc. The reaction mixture was dried <u>in vacuo</u>. The decyano-ethylated tetramer was reacted with 140 mg. of HO-TACT-OBz using the procedure of Example 6 to obtain 250 mg. (78%) of the title compound.

BAD ORIGINAL

## Claims

1. A process for selectively removing a β-cyanoethyl blocking group from the phosphate moiety of a nucleotide or polynucleotide, which comprises treating said nucleotide or polynucleotide with a primary amine having from 3 to 5 carbon atoms or diethylamine.

2. A process according to claim 1, in which the removal of the β-cyanoethyl group is carried out at a temperature of from 4°C. to 30°C.

3. A process according to claim 1 or 2, in which the removal of the β-cyanoethyl group is carried out at room temperature.

4. A process according to any one of claims 1 to 3, in which the removal of the β-cyanoethyl group is carried out in the presence of an inert organic solvent.

5. A process according to any one of claims 1 to 4, in which the removal of the β-cyanoethyl group is carried out in the presence of pyridine.

6. A process according to any one of claims 1 to 5, in which said nucleotide or polynucleotide is treated with <u>t</u>-butylamine.

7. A process according to any one of claims 1 to 5, in which said nucleotide or polynucleotide is treated with <u>sec</u>-butylamine.

8. A process according to any one of claims 1 to 5, in which said nucleotide or polynucleotide is treated with <u>n</u>-propylamine.

9. A process according to any one of claims 1 to 5, in which said nucleotide or polynucleotide is treated with diethylamine.

BAD ORIGINAL

10. A process according to any one of claims 1 to 9 in which at least a tenfold excess on a molar basis of a primary amine having 3 to 5 carbon atoms or diethylamine is present.

BAD ORIGINAL

European Patent Office

**EUROPEAN SEARCH REPORT**

0075392

Application number

EP 82 30 4434

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| | --- | | C 07 H 21/00 |
| Y | JOURNAL OF THE AMERICAN CHEMICAL SOCIETY, vol. 91, no. 12, 4th June 1969, pages 3350-3355 R.L. LETSINGER et al.: "Synthesis of oligothymidylates via phosphotriester intermediates" * Page 3351, left-hand column; page 3352, left-hand column * | 1 | |
| | --- | | |
| Y | TETRAHEDRON, vol. 34, 1978, pages 3143-3179, Pergamon Press Ltd., G.B. C.B. REESE: "The chemical synthesis of oligo- and poly-nucleotides by the phosphotriester approach" * Page 3158, left-hand column * | 1 | |
| | --- | | TECHNICAL FIELDS SEARCHED (Int. Cl. 3) |
| P,Y | EP-A-0 040 099 (ENS BIOLOGICALS INC.) * Page 41, claim 7 * | 1,6 | C 07 H 21/00 |
| | ----- | | |

The present search report has been drawn up for all claims

| Place of search | Date of completion of the search | Examiner |
|---|---|---|
| THE HAGUE | 26-11-1982 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82